# NEUE EUROPÄISCHE PATENTSCHRIFT

(11) **EP 1 062 916 B2**
(45) Veröffentlichungstag und Bekanntmachung des Hinweises auf die Entscheidung über den Einspruch: **18.09.2013**
(45) Hinweis auf die Patenterteilung: 10.01.2007
(21) Anmeldenummer: 00110016.3
(22) Anmeldetag: 12.05.2000
(51) Int. Cl.: A61C 13/00, A61C 5/10

(54) **Verfahren zur Herstellung eines individuell gefertigten, implantatgestützten Zahnersatzes und Verfahren zur Herstellung eines Zahnersatzteiles, insbesondere aus beliebigen, auch biokompatiblen Werkstoffen und insbesondere mit Hilfe der CAD-CAM-Fräs- und Schleif-Technik**
Process for manufacturing customized implant-mounted tooth replacements and process for making a dental prosthesis, especially of any, also biocompatible material and especially with the aid of the CAD-CAM method
Procédé de fabrication personnalisé de protheses dentaires fixées sur implants et procédé de fabrication d'une partie d'une prothèse dentaire, en particulier d'une quelconque matière, aussi en matière biocompatible, en particulier à l'aide de la méthode CAD-CAM, meulage et fraisage.

(30) Priorität: 21.06.1999 CH 115199
(43) Veröffentlichungstag der Anmeldung: 27.12.2000
(60) Teilanmeldung: 06018549.3
(73) Patentinhaber: Straumann Holding AG, 4002 Basel (CH)
(72) Erfinder: Traber, Tony, 4123 Allschwil (CH); Kappert, Heinrich F., Prof. Dr. rer. nat., 79184 Gundelfingen (DE); Gläser, Rainer, 79098 Freiburg i. Breisgau (DE)
(74) Vertreter: Schohe, Stefan

(56) Entgegenhaltungen:
- WO-A-98/44864
- DE-A- 19 616 618
- DE-A- 19 629 708
- DE-T- 69 413 852
- US-A- 5 184 306
- US-A- 5 347 454
- US-A- 5 401 170
- US-A- 5 527 182
- US-A- 5 816 810
- US-A- 5 857 853
- US-A- 5 873 721
- US-A- 5 873 721
- 'Curriculum, Prothetik, Band II', 1999, QUINTESSENZ VERLAGS-GMBH, BERLIN, ISBN 3876525284 vol. J.R.STRUB,J.C.TÜRP,S.WITKOWSKI,M.B.HÜRZELER ,M.KERN
- Ausschnitt aus Vorlesung von Professor Dr.Karl-Heinz Utz, Universität Bonn, Semester 1998/1999, "Zahnärztliche Versorgung des betagten Patienten", die am 14.10.1998 gehalten wurde; Mitschrift von Oliver Peusquens; Quelle http:/Jzahnmedizinstudenten.de/ download/inhalt/rubriken/klinik/prothetik/v orlesung/daten/981014.doc

## Beschreibung

Gegenstand der Erfindung ist ein Verfahren zur Herstellung eines Zahnersatzes nach dem Oberbegriff des Patentanspruchs 1, und wie in US-A-5 816 810 offenbart.

Unter dem Begriff 'Zahnersatz' soll im Rahmen der vorliegenden Beschreibung ein Gebilde verstanden werden, das nicht nur den sichtbaren Teil eines Zahnes oder einer Zahngruppe ergänzt oder ersetzt sondern auch dessen Zahnwurzel, und das somit einen vollständigen Ersatz für einen oder mehrere Zähne eines Patienten bildet. Dieser Zahnersatz besteht im gefertigten Zustand aus mehreren ineinandergreifenden oder auch aneinander befestigten Bestandteilen. Unter dem Begriff 'Zahnersatzteil' soll dagegen im Rahmen der vorliegenden Beschreibung ein Gebilde verstanden werden, das auf einem Implantat oder einem präparierten Zahnrest abgestützt wird und das im Wesentlichen den Kieferknochen und das Zahnfleisch überragt; ein Zahnersatzteil kann insbesondere einen Bestandteil eines implantatgestützten Zahnersatzes bilden.

Der erste Bestandteil des Zahnersatzes ist ein Implantat; welches im Kieferknochen des Patienten inseriert und dann auch als Implantat-Inserierung bezeichnet wird. Auf dieser Implantat-Inserierung werden später die weiteren Bestandteile des Zahnersatzes aufgebaut. Im Allgemeinen bildet die Implantat-Inserierung die Grundlage für einen Zahnersatz, der einen oder mehrere Zähne ersetzt; ein Zahnersatz, der einen Zahn ersetzen soll, wird auf einem einzigen Implantat abgestützt, und ein Zahnersatz, der mehrere benachbarte Zähne ersetzen soll, wird im Allgemeinen auf mindestens zwei Implantaten abgestützt. Die Implantate sind dazu bestimmt, vom Zahnarzt im Kieferknochen eines Patienten implantiert bzw. inseriert zu werden, und sie werden dem Zahnarzt gebrauchsfertig und steril geliefert. Die Implantate können im Wesentlichen zylindrisch oder zulaufend ausgebildet sein und weisen die Form von Schrauben oder Stiften auf. An seinem coronalen Ende weist jedes Implantat eine erstes Positionierungselement auf. Die Implantate sind im eingegliederten Zustand des Zahnersatzes am Patienten nicht oder nur bedingt sichtbar.

Der zweite Bestandteil des Zahnersatzes wird durch ein Verbindungsstück, das in der Fachsprache als Abutment bezeichnet wird, gebildet. An jedem Implantat wird ein Abutment befestigt. Die Abutments sind im eingegliederten Zustand des Zahnersatzes am Patienten nicht oder nur bedingt sichtbar. Sie dienen dazu, die Implantate mit den weiteren Bestandteilen des Zahnersatzes zu verbinden. Jedes Abutment weist an seinem proximalen Ende ein zweites Positionierungselement auf, das zusammen mit dem ersten, am zugehörigen Implantat angeordneten Positionierungselement eine gegen Rotation sichernde Positionierungsvorrichtung bildet.

Der dritte Bestandteil des Zahnersatzes wird durch Verbindungselemente zur gegenseitigen Befestigung von Implantat und Abutment, allgemein Schrauben, gebildet. Auch diese Verbindungselemente sind im eingegliederten Zustand des Zahnersatzes am Patienten nicht sichtbar. Die gegenseitige Verbindung von Implantat und Abutment ist im Allgemeinen reversibel.

Der vierte Bestandteil des Zahnersatzes wird als Gerüst, Brückengerüst oder Kappe bezeichnet. Er wird auf einem oder mehreren Abutments, im Allgemeinen mit Hilfe eines geeigneten Zementes oder Klebstoffes oder mittels einer Horizontal- oder Vertikalverschraubung gegen vertikale Verschiebungen gesichert beziehungsweise befestigt. Eine einzelne Kappe kann zum Ersatz eines Zahnes dienen und auf einem Abutment montiert werden. Ein Gebilde aus mehreren Kappen im Verbund mit einem Zwischenglied, als Pontic bezeichnet, kann aber auch als tragendes Element für eine Implantatbrücke zum Ersatz mehrerer Zähne bestimmt sein und auf mehreren Abutments montiert werden. Kappen sind zwar nach der Eingliederung des Zahnersatzes am Patienten nicht oder nur bedingt sichtbar, hingegen kann ihre Beschaffenheit einen gewissen Einfluss auf den ästhetischen Aspekt des Zahnersatzes ausüben, wie weiter unten dargelegt werden wird.

Der fünfte Bestandteil des Zahnersatzes wird durch eine Verblendung gebildet, welche das Gerüst beziehungsweise das Brückengerüst beziehungsweise die Kappe ummantelt. Die Verblendung ist der einzige Bestandteil des Zahnersatzes, der im eingegliederten Zustand des Zahnersatzes am Patienten sichtbar ist. Die Verblendung ist dazu bestimmt, den sichtbaren Bereich des Zahnes zu ersetzen. Das Gerüst beziehungsweise das Brückengerüst beziehungsweise die Kappe einerseits und die Verblendung anderseits sind irreversibel miteinander verbunden.

Anstelle des Gerüstes beziehungsweise des Brückengerüstes beziehungsweise der Kappe sowie der Verblendung, welche - wie eben beschrieben - den vierten und den fünften Bestandteil des Zahnersatzes bilden, kann auch eine sogenannte Vollkrone verwendet werden; in diesem Falle umfasst der Zahnersatz nur vier Bestandteile, nämlich das Implantat, das Abutment, die Verbindungselemente und die Vollkrone.

Gerüst und Verblendung zusammen oder die Vollkrone allein bilden eine Einheit, die im Rahmen der vorliegenden Beschreibung auch als Suprakonstruktion bezeichnet wird. Die Suprakonstruktion ist reversibel oder irreversibel am Abutment befestigt; reversibel befestigte Suprakonstruktionen können bei Bedarf vom Abutment entfernt werden.

Der Zahnersatz soll bezüglich Funktionalität, Form und Aussehen dem zu ersetzenden natürlichen Zahn beziehungsweise den zu ersetzenden natürlichen Zähnen des Patienten möglichst ähnlich sein.

Wie schon erwähnt sollen unter dem Begriff eines Zahnersatzteiles im Rahmen der vorliegenden Beschreibung Gebilde verstanden werden, die dazu bestimmt sind, unmittelbar oder mittelbar auf Implantaten oder Zahnresten befestigt zu werden. Einerseits fallen somit unter den Begriff eines Zahnersatzteiles Abutments, die auf Implantaten befestigt werden mit Gerüsten, die auf Abutments befestigt werden und mit Verblendungen, die auf den Gerüsten aufgetragen werden, ferner Abutments welche auch Gerüste bilden und Integralteile, welche alle Bestandteile des Zahnersatzes, mit Ausnahme des Implantates selbst, umfassen. Anderseits fallen unter den Begriff eines Zahnersatzteiles auch auf präparierten Zähnen befestigbare Teile wie beispielsweise kronen- und brückenartige Teile.

Die Herstellung von implantierbarem Zahnersatz kann in verschiedener Weise vor sich gehen, und auch der dabei entstehende Zahnersatz kann, wie oben beschrieben, unterschiedlich aufgebaut sein. Im Allgemeinen umfasst die Planung und Herstellung eines Zahnersatzes mehrere im Folgenden beschriebenen Verfahrensschritte, die teils vom Zahnarzt, teils vom Zahntechniker durchgeführt werden.

Der Aufgabenbereich des Zahnarztes beginnt mit der Erstellung eines Negativabdruckes, die auch als Kieferabformung bezeichnet wird. Die Erstellung eines Negativabdruckes bzw. einer Kieferabformungen ist ein Verfahrensschritt, bei welchem am Patienten Mass genommen wird, der aber am Patienten keine Spuren hinterlässt bzw. keine Veränderungen zur Folge hat. Aufgrund der Kieferabformung wird anschliessend durch den Zahntechniker ein Arbeitsmodell hergestellt; das Arbeitsmodell stellt die Situation im Kiefer des Patienten dar, in welchem der Zahnersatz zu integrieren ist. Der Zahnarzt liefert Angaben über die Anzahl der Implantate sowie gegebenenfalls weitere Angaben. Ferner inseriert der Zahnarzt die Implantate. Ein weiterer Negativabdruck wird nach der Inserierung der Implantate erstellt und liefert genauere Angaben für den Zahntechniker, die weiter unten beschrieben werden. Nach der vorgegebenen erforderlichen Heilphase, welche auf die Inserierung der Implantate im Kiefer des Patienten folgt, legt der Zahnarzt die Implantate beziehungsweise deren äusserste Bereiche frei. Die optimale Lage der Implantate ist für die Form der weiteren Bestandteile in Bezug auf Okklusion, Funktion und Ästhetik entscheidend; beim Inserieren der Implantate im Kiefer des Patienten ist es leider nicht immer möglich, die Implantate entsprechend den Idealvorstellungen bezüglich Okklusion, Funktion und Ästhetik optimal anzuordnen; die Folge davon ist, dass der Zahnersatz im Mund des Patienten bezüglich Okklusion, Funktion und Ästhetik häufig nicht optimal zu erstellen ist. Als letztes montiert der Zahnarzt auf den lmplantaten nach einiger Zeit, während welcher diese einwachsen, die restlichen Bestandteile des Zahnersatzes.

Der Zahntechniker beginnt, wie oben erwähnt, mit der Erstellung eines Arbeitsmodells, des sogenannten Arbeitsmodelles, das aufgrund des vom Zahnarzt gelieferten Negativabdruckes bzw. der Kieferabformung erstellt wird. Im weiteren Verfahren wird stets das Arbeitsmodell benutzt; auf dem Arbeitsmodell erfolgen die wesentlichen Arbeitsgänge der Herstellung des Zahnersatzes.

Der Zahntechniker arbeitet Im Arbeitsmodell je nach herzustellendem Zahnersatz ein oder mehrere Manipulierimplantate, die auch als Modellanalog bezeichnet werden, ein. Die Manipulierimplantate sind kein Bestandteil des zu erstellenden Zahnersatzes sondern lediglich Hilfen zur Erstellung des Zahnersatzes. Das Modellanalog bildet die Grundlage für die Anfertigung der restlichen Bestandteile des Zahnersatzes. Die Position der Manipulierimplantate ist durch die vom Zahnarzt gelieferte Kieferabformung mit den Implantaten festgelegt. Die präzise Darstellung der Positionen der Implantate ist für die weitere Herstellung des Zahnersatzes entscheidend.

Auf jedes am Arbeitsmodell angeordnete Modellimplantat wird ein Abutment aufgebracht. Für die Beschaffung geeigneter Abutments gibt es mehrere Möglichkeiten. Es können vorkonfektionierte Abutments verwendet werden, was eine preisgünstigere und weniger zeitraubende Möglichkeit als die Herstellung indivudueller Abutments ist. Individuelle Abutments können durch einen Giessvorgang hergestellt werden, was aber mit mehreren Nachteilen behaftet ist; insbesondere ist es bei Brücken- und ähnlichen Konstruktionen schwierig, eine präzise Form sowie ein spannungsfreies Gebilde herzustellen; des Weiteren besteht bem Giessen die Gefahr von temperaturbedingten Werkstoffveränderungen. Individuelle Abutments können als Einzelteile individuell gefertigt werden, wobei sie individuell entweder aus einem Rohling oder aus einer Platte konfektioniert werden. Schliesslich können zur Hersztellung individueller Abutments auch konfektionierte Abutments verwendet werden, die nachbearbeitet beziehungsweise individualisiert werden, wozu sie auf einer plattenartigen Haltevorrichtung befestigt werden. Die Herstellung individualisierter Verbindungskörper ist also in jedem Falle aufwendig.

Ein grosser Zeitaufwand entsteht bei der Herstellung implantatgetragener Brücken, und zwar im Zusammenhang mit dem Einschieben der Suprakonstruktion auf die Implantate und die Abutments. Während der Einschubvorgang bei der Herstellung eines kronenartigen Zahnersatzes, weicher sich über den Bereich nur eines Zahnes erstreckt, nicht mit allzu vielen Problemen behaftet ist, ist er bei der Erstellung eines brückenartigen Zahnersatzes, der sich über den Bereich mehrere Zähne erstreckt, bedeutend problematischer; da sich die Suprakonstruktion dabei mittelbar auf mehrere Implantate und unmittelbar auf mehrere Abutments abstützt. Die Implantate beziehungsweise deren Längsachsen sind im Allgemeinen nicht parallel sondern divergierend angeordnet und erfordern damit entsprechend divergierende Einschubrichtungen. Eine in einem Stück hergestellte Suprakonstruktion in Brückenform kann aber natürlich nur in einer einzigen Einschubrichtung eingegliedert werden, das heisst die Einschubrichtungen auf mehrere Implantate beziehungsweise Abutments müssten parallel sein, was sich derzeit nur unter erschwerten Bedingungen realisieren lässt. Die Eingliederung wird bei grossen Abweichungen unmöglich, oder erst dann möglich, wenn eine manuelle, grossen Aufwand erfordernde Nachbearbeitung stattgefunden hat. Bei kleineren Abweichungen ist es noch möglich, die Eingliederung im Munde des Patienten vorzunehmen, allerdings nur unter Erzeugung von Spannungen an den Implantaten und/oder an den Abutments und/oder an der Suprakonstruktion

Mit den herkömmlichen Verfahren, die mit vielen möglichen Fehlerquellen behaftet sind, ist es derzeit ausserordentlich schwierig, eine spannungsfreie Konstruktion mit nicht veränderten Werkstoffeigenschaften des Zahnersatzes zu erhalten.

Aufgabe der Erfindung ist es daher,
- ein verbessertes Verfahren zur Herstellung eines individuellen implantatgestützten Zahnersatz vorzuschlagen.

Die Lösung dieser Aufgabe erfolgt
- für die Herstellung des Zahnersatzes durch die Merkmale des kennzeichnenden Teils des Patentanspruchs 1.

Vorteilhafte Weiterbildungen der erfindungsgemässen Verfahren werden durch die abhängigen Patentansprüche 2-11 definiert.

Zur Herstellung eines Zahnersatzes wird wie bei herkömmlichen Verfahren vom Zahnarzt ein Negativabdruck bzw. eine Negativabformung des Kiefers oder Kieferbereiches, in welchen der anzufertigende individuelle Zahnersatz später zu liegen kommt, hergestellt, um lnformationen bezüglich der Lage der Basis beziehungsweise des Implantates oder der Implantate zu bekommen. Vom Zahntechniker wird aufgrund dieser Negativabformung ein Arbeitsmodell hergestellt. Anschliessend wird auf dem Arbeitsmodell eine Manipulierimplantatanordnung angebracht, die bezüglich ihrer Lage den Implantaten im Mund des Patienten entspricht. Die Manipulierimplantatanordnung kann aus einem oder mehreren Manipulierimplantaten bestehen, entsprechend der Anzahl der tatsächlichen Implantate. Die Manipulierimplantate ragen nicht oder kaum aus dem Arbeitsmodell, ebenso wie die Implantate nicht oder kaum aus dem entsprechenden Kieferbereich des Patienten ragen. Als nächstes wird daher auf jedem Manipulierimplantat provisorisch ein Hilfselement angebracht. Diese Hilfselemente ragen aus dem Arbeitsmodell und bilden Hilfselemente bzw. Vermessungsteile, da sie die Einsatztiefe, die Längsachsenrichtung und die Winkelstellung der Manipulierimplantate im Arbeitsmodell und damit auch die Implantat-Tiefe, die Implantat-Achsrichtung und die Implantat-Winkellage im Mund des Patienten wiedergeben. Mit Hilfe dieser Hilfselemente oder Vermessungsteile lassen sich anschliessend Daten der Geometrie des Arbeitsmodelles erfassen, und daraus kann auf die genaue Lage der Implantate im Kieferbereich des Patienten geschlossen werden. Aus den einmal erfassten Daten lassen sich weitere Daten ermitteln, die für die vollautomatisierte Herstellung sowie für die Ermittlung der Einschubrichtung der weiteren Bestandteile des individuellen Zahnersatzes benötigt werden; insbesondere ist es möglich, die Bestandteile von mehrere Implantate erfassendem Zahnersatz so herzustellen, dass die Eingliederung in einer einzigen Einschubrichtung, die auch als parallele Einschubrichtung bezeichnet wird, erfolgen kann. Dadurch wird in jeder Beziehung eine bisher unerreichte Präzision gewährleistet.

Die erfindungsgemässe Herstellung eines Zahnersatzteiles nach dem neuen Verfahren ist im wesentlichen gleich wie die Herstellung eines Zahnersatzteiles, mit Ausnahme der Verfahrensschritte, die sich lediglich auf die Implantate beziehen, da Zahnersatzteille entweder auf vorhandene Restzähne oder auf als vorhanden vorausgesetzte Implantate abgestützt sind.

Weitere Einzelheiten und Vorteile der Erfindung werden im folgenden anhand von Ausführungsbeispielen und mit Bezug auf die Zeichnung beschrieben. Es zeigen:
- **Fig. 1**: einen Zahnersatz nach der Erfindung, in einem die Längsachse des Zahnersatzes enthaltenden Schnitt;
- **Fig. 2**: einen Kieferbereich eines Patienten, vor der Implantierung der Implantate, in einem Schaubild;
- **Fig. 3**: ein Arbeitsmodell des in ***Fig. 2*** dargestellten Kieferbereiches, ausschnittweise, mit zwei divergent stehenden Implantaten und einem Hilfselement pro Implantat, vereinfacht dargestellt, in einem Schnitt;
- **Fig. 4**: das in **Fig. 3** dargestellte Arbeitsmodell, mit auf den divergent stehenden Implantaten angeordneten Abutments, wobei die Abutments bezüglich einer gemeinsamen Einschubrichtung modifiziert sind, in gleicher Darstellung wie ***Fig. 3***;
- **Fig. 5**: den in ***Fig. 2*** dargestellten Kieferbereich des Patienten mit einem aufgrund des Arbeitsmodells der ***Fig. 3*** und der ***Fig. 4*** hergestellten Zahnersatz, in gleicher Dastellung wie ***Fig. 3*** und ***Fig. 4***;
- **Fig. 6**: eine Standardplattte, dargestellt mit Einzelkronen und einem Brückengerüst;

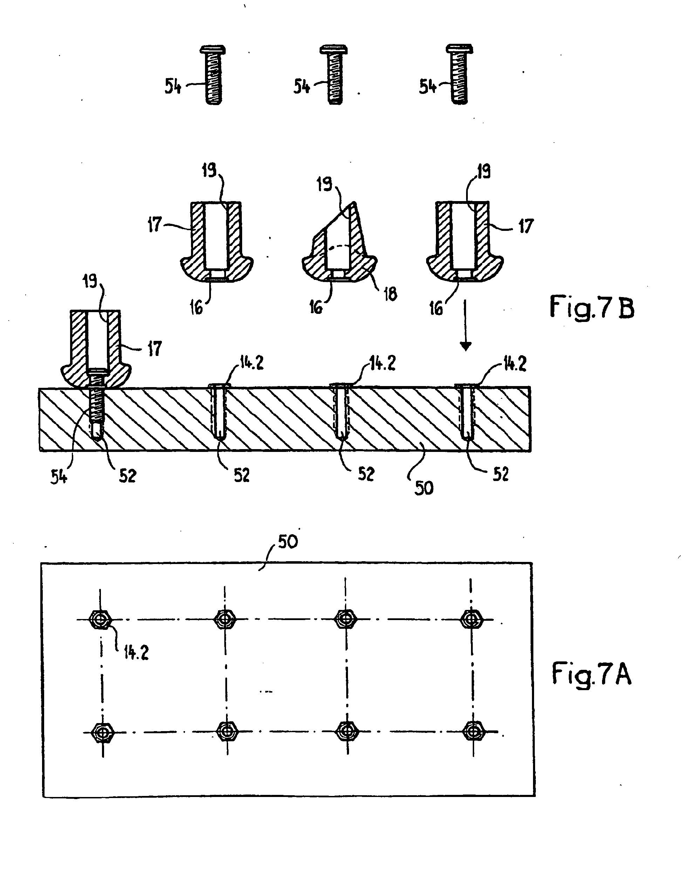

Der gegenstand der Figuren 7-10 ist kein Teil der Erfindung
- **Fig. 7A**: eine Platte zur Aufnahme von Abutment-Rohteilen, auf welcher mehrere Abutments während ihrer Bearbeitung fixierbar sind, in Draufsicht;
- **Fig. 7B**: die in ***Fig. 7A*** dargestellte Platte mit einem auf der Platte befestigten Abutment-Rohteil und mit drei nicht befestigten Abutments sowie der zugehörigen Befestigungsschrauben, in einem Schnitt senkrecht zur Plattenhauptebene;

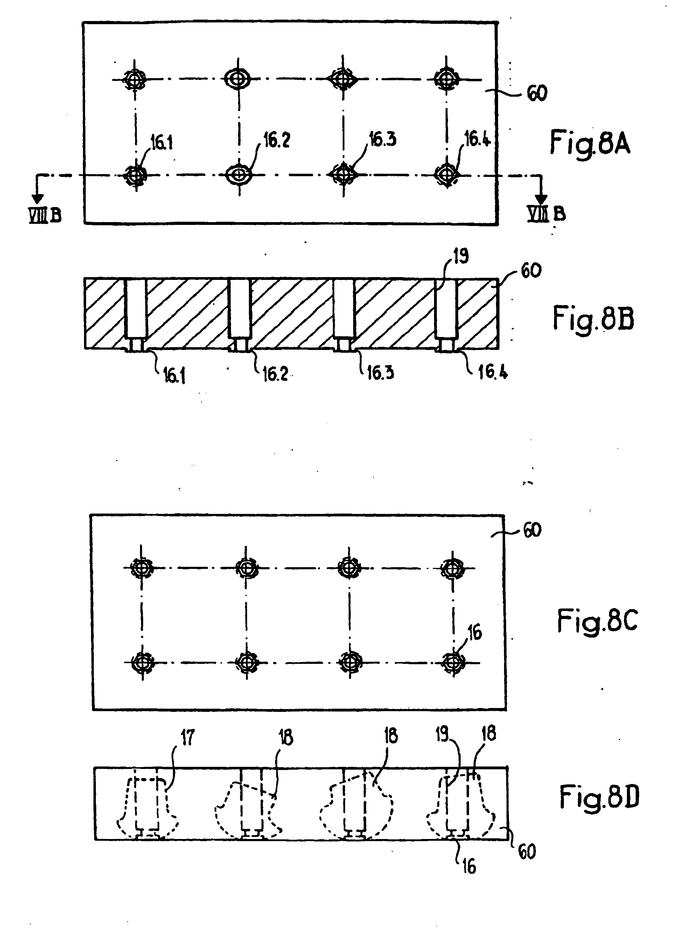

- **Fig. 8A**: eine Platte, aus welcher anatomisch korrekte Abutments korrekt fräsbar sind, in Draufsicht;
- **Fig. 8B**: die in ***Fig. 8A*** dargestellte Platte, in einem Schnitt senkrecht zur Plattenhauptebene;
- **Fig. 8C**: eine weitere Platte, aus der Abutments herstellbar sind, in gleicher Darstellung wie ***Fig. 8A***;
- **Fig. 8D**: die in ***Fig. 8C*** dargestellte Platte, in welcher die Formen der herzustellenden Abutments in gestrichelten Linien dargestellt sind, von der Seite;

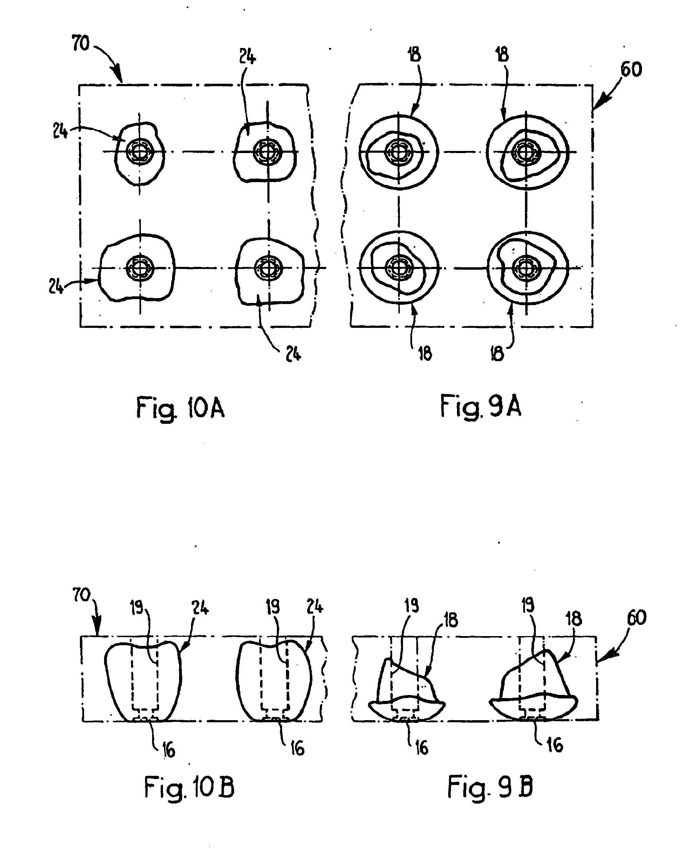

- **Fig. 9A**: vier aus einer Platte hergestellte Abutments in derjenigen Lage, die sie in der Platte eingenommen haben, wobei die Platte gestrichelt dargestellt ist, in Draufsicht;
- **Fig. 9B**: die in ***Fig. 9A*** dargestellten Abutments, wobei die Platte, aus welcher sie hergestellt sind, gestrichelt dargestellt ist, von der Seite;
- **Fig. 10A**: vier aus einer Platte hergestellte aus der Integralteile, umfassend Abutments, Gerüste und Verblendungen, in derjenigen Lage, die sie in der Platte eingenommen haben, wobei die Platte gestrichelt dargestellt ist, in gleicher Darstellung wie ***Fig. 9A******;***
- **Fig. 10B**: die in ***Fig. 10A*** dargestellten Integralteile, in gleicher Darstellung wie ***Fig. 9B***;

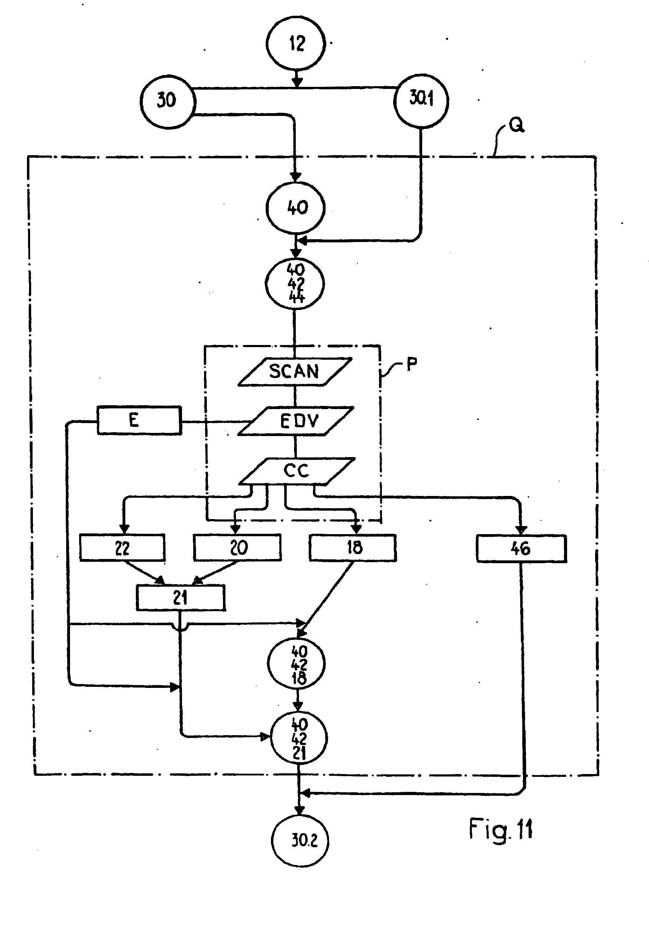

- **Fig. 11**: ein Blockdiagramm zur Verdeutlichung des erfindungsgemässen Verfahrens zur Herstellung eines Zahnersatzes; und

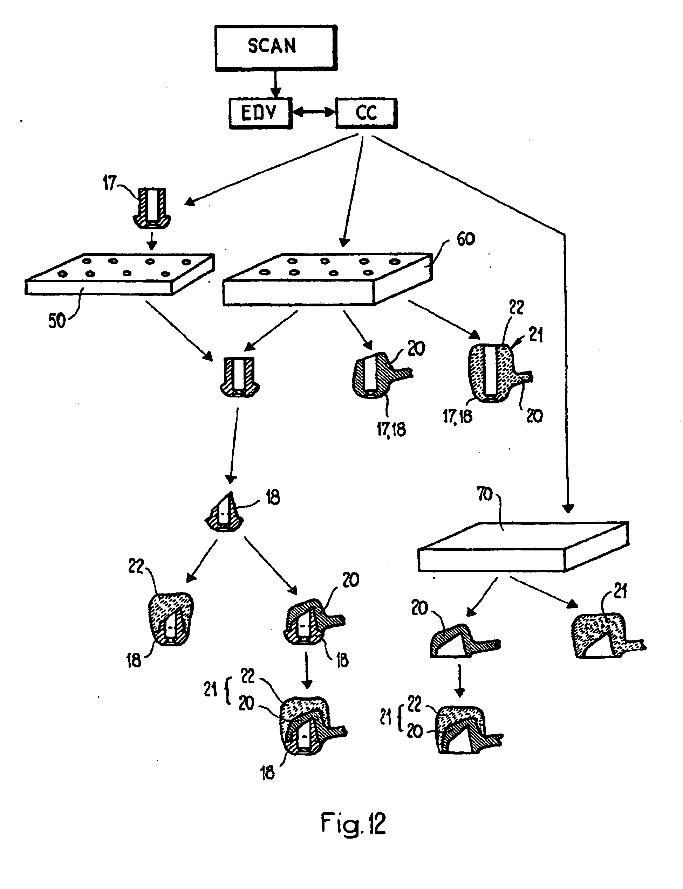

- **Fig. 12**: ein Schema zur Erläuterung unterschiedlicher Herstellungsmethoden und -phasen von Zahnersatzteilen.

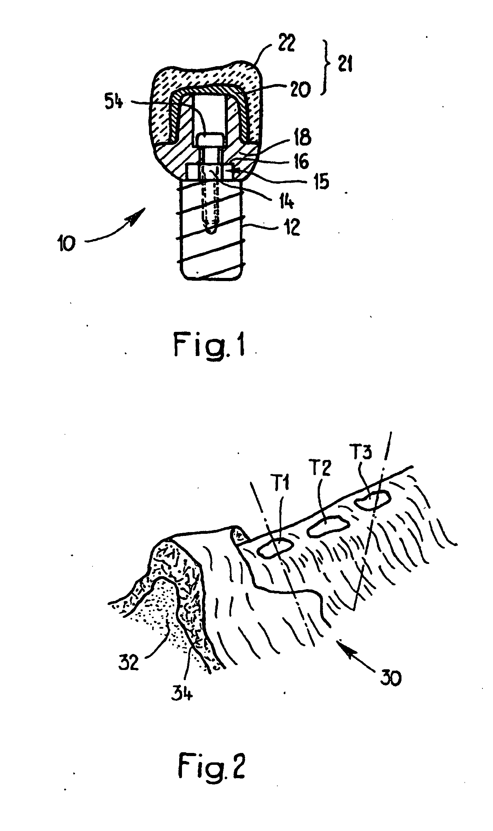

In **Fig. 1** ist ein vollständiger individueller Zahnersatz **10** dargestellt. Der individuelle Zahnersatz **10** umfasst ein Implantat **12**, welches eine Basis für weitere Bestandteile des individuellen Zahnersatzes **10** bildet. Das Implantat **12** ist als hier als Schraube ausgebildet, kann aber auch als Rohr oder Stift ausgebildet sein. Das Implantat **12** ist dazu bestimmt, als Ersatz für die Zahnwurzel im Kieferknochen des Patienten befestigt zu werden. An seinem in **Fig. 1** oberen Ende weist das Implantat **12** ein erstes Positionierungselement **14** einer Positionierungsvorrichtung **15** auf. Ein zweites, komplementär zum ersten abgebildeten Positionierungselement **16** derselben Positionierungsvorrichtung **15** ist an einem Abutment **18** angeordnet und in **Fig. 7A, 7B** dargestellt. Ebenfalls ist in **Fig. 7B** eine Durchgangsbohrung **19**, die auch als Montagekanal bezeichnet wird, sichtbar, die zur Aufnahme eines Befestigungselementes wie einer Schraube **54** vorgesehen ist, mittels welcher das Abutment **18** auf dem Implantat **12** befestigt ist. Ein jeweils geeignetes Implantat **12** wird aus einer Reihe verschiedener serienmässig hergestellter Implantate ausgewählt und ohne weitere Bearbeitung durch den Zahnarzt im Kieferknochen des Patienten implantiert. Auf dem Abutment **18** ist gemäss **Fig. 1** ein Gerüst **20** aufgebaut, das auch als Kappe bezeichnet wird. Für das Gerüst **20** können unterschiedliche Werkstoffe und Herstellungstechniken verwendet werden, wie dies weiter unten beschrieben wird. Auf dem Gerüst **20** ist eine Verblendung **22** befestigt, welche zusammen mit dem Gerüst **20** eine im Rahmen der vorliegenden Beschreibung eine als Suprakonstruktion **21** oder Mesiostruktur **21** bezeichnete Einheit des individuellen Zahnersatzes **10** bildet. Die Verblendung **22** stellt im Wesentlichen den Ersatz für den sichtbaren Teils des Zahnschmelzes dar. **Fig. 1** dient nur zur Erläuterung der Bestandteile, aus welchen der im Munde des Patienten angebrachte individuelle Zahnersatz **10** aufgebaut ist, nicht aber zur Beschreibung des Vorgehens beim Aufbau beziehungsweise bei der Montage des individuellen Zahnersatzes **10**.

**Fig. 2** stellt ausschnittweise einen Kieferbereich **30** mit einem Kieferknochen **32** und Zahnfleisch **34** eines Patienten dar, der mit einem brückenartigen individuellen Zahnersatz **10** zu versehen ist, und zwar an den Stellen **T1, T2, T3,** wo ursprünglich drei benachbarte Zähne angeordnet waren. An diesem Kieferbereich **30** wird vom Zahnarzt eine nicht dargestellte Kieferabformung in Form eines Negativmodelles beziehungsweise Negativabdruckes angefertigt. Ferner wird der Zahnarzt an den Stellen **T1** und **T3** je ein in **Fig. 2** nicht dargestelltes Implantat **12** implantieren beziehungsweise inserieren und auf diesen beiden Implantaten **12**, sobald sie ossio-integriert sind. die weiteren Bestandteile des individuellen Zahnersatzes **10** befestigen, die vom Zahntechniker hergestellt werden. Ausserdem liefert der Zahnarzt Informationen bezüglich der Lage der beiden Implantate **12** und der erwünschten Ausbildung der weiteren Bestandteile des individuellen Zahnersatzes **10**.

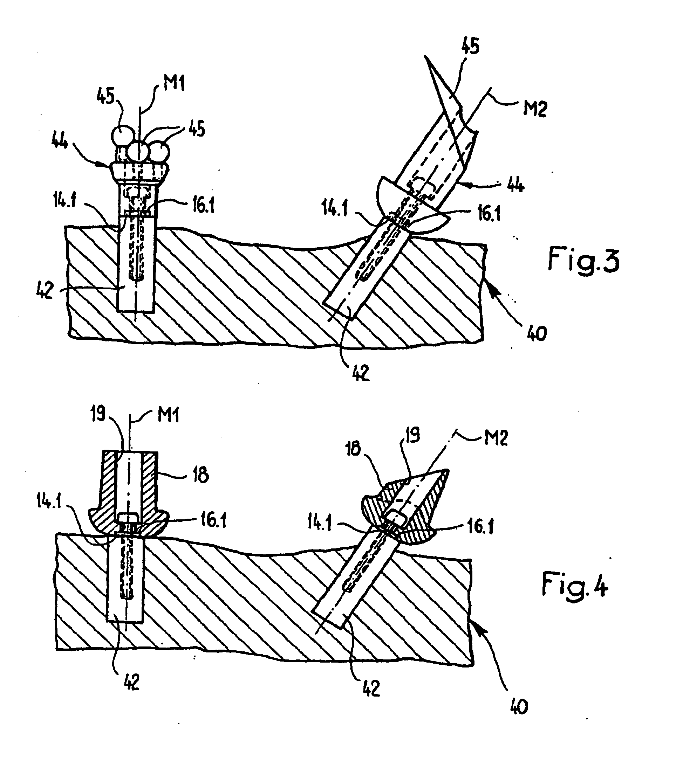

Aufgrund des Negativabdruckes beziehungsweise der Kieferabformung des Zahnarztes werden vom Zahntechniker eines oder mehrere Arbeitsmodelle, insbesondere ein in **Fig. 3** ausschnittweise wiedergegebenes Arbeitsmodell **40**, hergestellt. Das Arbeitsmodell **40** dient dem Zahntechniker als Grundlage zur Herstellung des individuellen brückenartigen Zahnersatzes **10**, der die drei ursprünglich bei **T1, T2** und **T3** vorhandenen gewesenen Zähne ersetzen soll. Der brückenartige individuellen Zahnersatz **10** weist als Basis, wie schon erwähnt, die zwei Implantate **12** auf. Am Arbeitsmodell **40** werden nun zwei Manipulierimplantate **42** befestigt, und zwar an Stellen bzw. mit Längsachsen **M1** beziehungsweise **M2**, die den Stellen bzw. Hauptrichtungen **T1** und **T3** von zweien der zu ersetzenden drei Zähne möglichst genau entsprechen.

In **Fig. 3** ist diejenige Herstellungsphase des individuellen Zahnersatzes **10** dargestellt, in welcher auf jedem der Manipulierimplantate **42** ein provisorisch zugehöriges Hilfselement beziehungsweise Vermessungsteil **44** angeordnet ist; diese Hilfselemente beziehungsweise Vermessungsteile **44** gehören nicht zum definitiven Zahnersatz **10**. Die Hilfselemente beziehungsweise Vermessungsteile **44** weisen Enden bzw. Markierungen **45** auf, anhand derer sich ihre winkelmässige Anordnung erkennen lässt. Die beiden Manipulierimplantate **42** beziehungsweise ihre Längsachsen stehen häufig weder parallel noch liegen sie in einer gemeinsamen Ebene, sondern sie nehmen divergierende Stellungen ein.

An jedem Manipulierimplantat **42** wird in einer späteren Herstellungsphase des individuellen Zahnersatzes **10** gemäss **Fig. 4** ein Abutment **18** provisorisch montiert. Das Abutment **18** weist an seinem dem Implantat zugewandten Ende das Positionierungselement **16** auf, welches letztlich dazu bestimmt ist, mit dem am oberen beziehungsweise äusseren Ende des Implantates **12** vorhandenen komplementären Positionierungselement **14** zusammenzuwirken, um mit dem letzteren zusammen die zur Rotationsverhinderung dienende Positionierungsvorrichtung **15** zu bilden. Entsprechend weisen das Manipulierimplantat **42** ein Positionierungselement **14.1** und das Hilfselement beziehungsweise Vermessungsteil **44** ein Positionierungselement **16.1** auf, welches eine relative Rotation verhindert und die Position sichert.

Der entsprechende Bereich des Arbeitsmodells **40** mit den sichtbaren Teilen der Hilfselemente beziehungsweise Vermessungsteile **44,** jedoch ohne die Abutments **18,** in dreidimensionaler Konfiguration wird mittels einer dreidimensionalen Erfassungsanlage, vorzugsweise mittels eines Scanners **SCAN,** der schematisch in **Fig. 10** wiedergegeben ist, erfasst und die entsprechenden Werte in einer Speichereinheit einer EDV-Anlage **EDV** gespeichert beziehungsweise gelagert Die durch den Scan-Vorgang ermittelten Werte werden anschliessend mit Hilfe der EDV-Anlage **EDV**, welche auch in einer CAD- beziehungsweise CAM- beziehungsweise CAD/CAM-Anlage **CC** integriert sein kann, in Basis-Daten **BAD** umgesetzt. Diese Basis-Daten **BAD** werden für alle weiteren Berechnungen und automatisierten Bearbeitungsvorgänge benutzt.

Aus den Basis-Daten **BAD** werden Implantat-Daten **ID** ermittelt, welche aufgrund der Lage der Hilfselemente beziehungsweise Vermessungsteile **44** im Arbeitsmodell **40** die Lage der Implantate **12** im Kieferbereich **30** des Patienten beschreiben. Aus den Basis-Daten **BAD**, die die Lage der Hilfselemente **44** definieren, lassen sich somit die Neigung, die Implantierungstiefe und die Stellung der Implantate **12** selbst sowie insbesondere die Ausrichtung des Positionierungselements **14,** beispielsweise Hex, etc., ermitteln.

Aus den Implantat-Daten **ID** sowie aus weiteren Daten, die auch durch ein Wax-Up gewonnen werden können, lassen sich Abutment-Daten **AD** gewinnen, welche die Formgebung und die beabsichtigte Lage des beziehungsweise der für den jeweiligen Fall erforderlichen Abutments **18** definieren. Aufgrund dieser Abutment-Daten **AD** wird ein geeigneter Verfahrensablauf bzw. ein geeignetes Flussverfahren gewählt. Anschliessend wird in der CAD/CAM-Anlage **CC** das individuelle Abutment **18** durch spanabhebende Bearbeitung wie Schleifen und/oder Fräsen hergestellt, wobei als Rohmaterial entweder ein einzelnes Abutment-Rohteil oder eine Platte aus Abutmentmaterial verwendet wird, wie dies weiter unten beschrieben wird. Die vollautomatisierte Fertigung der Abutments **18** auf der CAD/CAM-Anlage **CC** gewährleistet nicht nur die geometrische Passgenauigkeit sondern auch die Einhaltung der vorbestimmten Materialeigenschaften, da diese während des Bearbeitungsprozesses keinen unvorhersehbaren mechanischen und thermischen Einflüssen ausgesetzt werden.

Auf das Verfahren zur Herstellung der individuellen Abutments wird weiter unten genauer eingegangen.

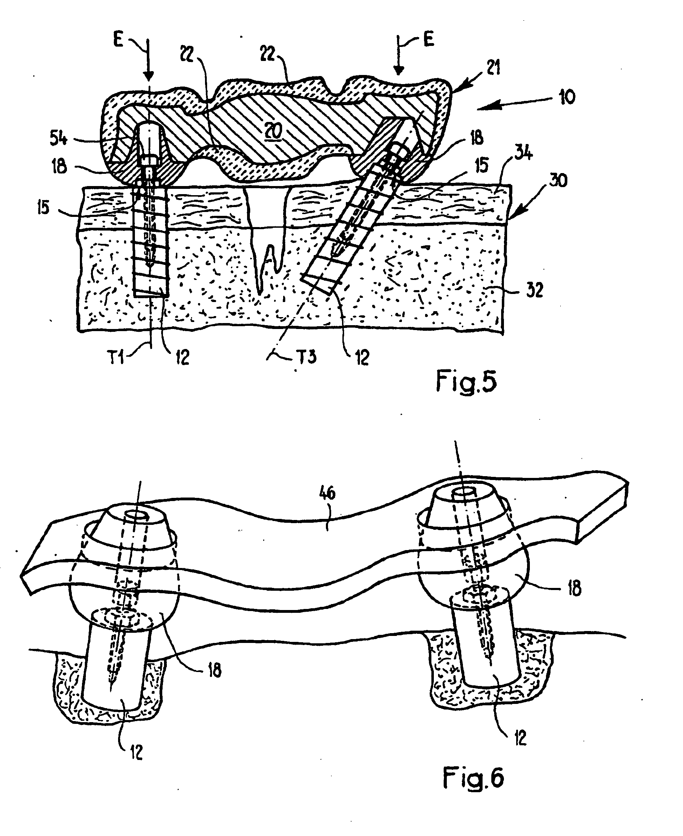

Werden gemäss den **Fig. 2** bis **6** mehrere Abutments **18** für den individuellen Zahnersatz **10** benötigt, der in fertigem, am Patienten montiertem Zustand in **Fig. 5** dargestellt ist, so müssen sie derart konfiguriert sein, dass die tiefen-, richtungs- und winkelmässigen Abweichungen zwischen den Implantaten **12** kompensiert werden, damit anschliessend die Suprakonstruktion **21**, bestehend aus dem Gerüst **20** und der Verblendung **22**, in einer gemeinsamen Einschubrichtung montiert werden kann, ohne dass Spannungen in den Implantaten **12**, in den Abutments **18** und in der Suprakonstruktion **21** entstehen. Zu diesem Zwecke kann die EDV-Anlage **EDV** aus den Basis-Daten **BAD** nicht nur die eigentlichen Abutment-Daten **AD** sondern auch Einschub-Daten **ED** ermitteln, welche die Einschubrichtung, insbesondere bei mehreren Implantaten **12** die gemeinsame Einschubrichtung, definieren, in welcher schliesslich die Suprakonstruktion **21** auf die Abutments **18** geschoben wird.

**Fig. 6** zeigt einen Kieferbereich mit zwei Implantaten **12** und je einem darauf befestigten Abutment **18**, sowie mit einer Standardplatte **46** für eine Brückenkonstruktion.

Die EDV-Anlage kann aus den Basis-Daten **BAD** ferner Gerüst-Daten **GD** bestimmen, da im Wesentlichen die innere seitliche Fläche des Gerüstes **20** auf die äussere bzw. obere Fläche der Abutments **18** passen muss, damit das Gerüst **20** spannungsfrei und haltbar auf den Abutments **18** befestigt werden kann. Dies bedeutet nicht, dass die beiden genannten Flächen kongruent sein müssen, da zu ihrer gegenseitigen Befestigung Zement verwendet wird, für welchen ein geringer Spalt freizuhalten ist. Auch das Gerüst **20** oder die kronen- oder brückenartige Suprakonstruktion **21** kann mit Hilfe der CAD/CAM-Anlage **CC** hergestellt werden, wobei sich dieselben Vorteile ergeben, die weiter oben bezüglich der Herstellung der Abutments **18** erwähnt worden sind.

Der individuelle Zahnersatz **10,** jedoch ohne die ihn später im Kiefer des Patienten tragenden Implantate, wird provisorisch am Arbeitsmodell **40** aufgebaut; anstelle der Implantate **12** werden hierbei die Manipulierimplantate **42** benutzt. Anschliessend erfolgt der Transfer der Abutments **18** sowie des Gerüstes **20** und der Verblendung **22** in den Kieferbereich **30** des Patienten; dieser Kieferbereich mit dem fertigen individuellen Zahnersatz ist in **Fig. 5** dargestellt. Die Abutments **18** werden dabei auf den Implantaten **12** mittels der Positionierungselemente **16** positioniert und mittels Schrauben befestigt. Auf die Abutments **18** wird dann die Suprakonstruktion **21,** mit dem Gerüst **20** und der Verblendung **22**, geschoben, und zwar in der Einschubrichtung **E,** die für die verschiedenen Abutments **18** parallel ist.

Wesentlich für die Präzision des individuellen Zahnersatzes **10** bei der Herstellung ist, dass zu allen Zwecken, zu denen Daten benutzt werden müssen, stets auf dieselben, durch einen einzigen Scan-Vorgang ermittelten Basis-Daten **BAD** zurückgegriffen wird, aus welchen sich unmittelbar und mittelbar alle weiteren Daten wie zum Beispiel Abutment-Daten **AD**, Fixierungsdaten **FD**, Gerüstdaten **GD**, Verblendungsdaten **VD** ableiten lassen, um sie für die Konfiguration und Bearbeitung der verschiedenen Bestandteile in der CAD/CAM-Anlage **CC** zu benutzen. Ebenso wichtig ist es, dass keine manuellen Verfahrensschritte erforderlich sind, welche die präzise Position der Implantate **12** und die Lage, Form und Materialbeschaffenheit der Abutments **18** sowie die Lage der Suprakonstruktion **21** beeinflussen. Die Basis-Daten **BAD** ermöglichen ein systematisches, kohärentes Verfahren zum Aufbau des individuellen Zahnersatzes.

Im folgenden wird auf Einzelheiten der Herstellung des Abutments **18** und auf Abutment-Rohteile **17** eingegangen. Als Abutment-Rohteil **17** für ein einzelnes Abutment **18** wird ein Rohling oder ein Halbfabrikat verwendet, welches vom Hersteller dazu bestimmt ist, nachgearbeitet und dadurch individualisiert zu werden.

Eine Bibliothek in der Speichereinheit der EDV-Anlage **EDV** kann die Daten von erhältlichen vorgefertigten Abutment-Rohteilen **17** enthalten, wobei es sich hierbei nicht nur um Abutment-Rohteile **17** aus einem einzigen Material oder von einem einzigen Hersteller handeln muss. Aus diesen erhältlichen vorgefertigten Abutment-Rohteilen **17** sucht die EDV-Anlage **EDV** nun für jedes Implantat **12** das zugehörige Abutment-Rohteil **17** aus. Im Allgemeinen handelt es sich beim jeweils geeignetsten Abutment-Rohteil **17** um dasjenige, aus welchem sich das passende Abutment **18** mit dem geringsten Bearbeitungsaufwand fertigen lässt. Im weiteren bestimmt die EDV-Anlage **EDV** alle geometrischen Bearbeitungsdaten sowie beispielsweise Schnitt- und Vorschubgeschwindigkeiten, Menge des benötigten Kühlmittels und andere Daten, nach welchen die Bearbeitungseinheit der CAD/CAM-Anlage **CC** das Abutment 18 aus dem Abutment-Rohteil **17** herstellt.

Alternativ kann als Abutment-Rohteil **17** auch ein konfektioniertes Norm-Abutment verwendet werden, das zwar vom Hersteller als fertiges Abutment angeboten wird und das ohne weitere Bearbeitung zur sofortigen Verwendung bestimmt wäre; ein solches Norm-Abutment muss aber zur Erzeugung eines optimalen individuellen Zahnersatzes **10** dennoch weiterbearbeitet und dadurch individualisiert werden.

Einzelheiten zur Herstellung der Abutments **18** aus den Abutment-Rohteilen **17** werden in **Fig. 7A** und **7B** gezeigt. Die Abutment-Rohteile **17** werden während ihrer Bearbeitung auf einer Vorrichtung befestigt. Dabei können Vorrichtungen verwendet werden, die zur Aufnahme eines einzelnen Abutment-Rohteiles **17** bestimmt sind. Es hat sich aber als günstig erwiesen, mehrere Abutment-Rohteile auf einer gemeinsamen Vorrichtung zu befestigen. Diese Vorrichtung in Form einer Platte **50** ist in **Fig. 7A** in Aufsicht dargestellt. Auf dieser Platte **50** sind zum Beispiel an acht Stellen, nämlich in zwei Reihen an je vier Stellen, maximal acht in **Fig. 7A** nicht dargestellte Abutment-Rohteile **17** montierbar. Die Platte **50** weist an jeder der acht Stellen ein Positionierungselement **14.2** auf, das dem ersten Positionierungselement **14** des Implantates **12** beziehungsweise dem Positionierungselement **14.1** des Manipulierimplantates **42** entspricht und das dazu bestimmt ist, mit dem Positionierelement **16** am Abutment-Rohteil **17** bzw. am Abutment **18** zusammenzuwirken. Ferner enthält die Platte **50** an jeder der acht Stellen eine nur in **Fig. 7B** sichtbare Gewindebohrung **52**. **Fig. 7B** stellt die Platte **50** der **Fig. 7A** im Schnitt dar, jedoch mit einem mittels einer Schraube **54** auf der Platte **50** befestigten Abutment-Rohteil **17** sowie mit zwei mittels weiterer Schrauben **54** auf der Platte **50** befestigbaren Abutment-Rohteilen **17** und mit einem aus einem bearbeiteten Abutment-Rohteil entstandenen, durch Lösung der Schraube **54** von der Platte **50** entfernten Abutment **18**. Solche Platten **50** sind bekannt. Die bisher auf ihnen hergestellten Abutments wiesen aber Achsen auf, die relativ zur Platte **50** parallel waren, während es in Wirklichkeit erforderlich ist, dass die verschiedenen Abutments relativ zur Platte **50** unterschiedlich gerichtete Achsen besitzen; die Richtungen dieser Achsen werden nach dem neuen Verfahren durch Verwendung der Hilfselemente beziehungsweise Vermessungsteile **44** durch die Erfassungseinrichtung **SCAN** festgestellt und bei der Herstellung der Abutments **18** mittels der CAD/CAM-Anlage **CC** berücksichtigt, so dass die derart erzeugten Abutments **18** achskonform montierbar sind.

Eine weitere Möglichkeit zur Herstellung von Abutments ist in **Fig. 8A, 8B, 8C** und **8D** dargestellt. Hierbei werden die Abutments **18** direkt aus einer Platte **60** hergestellt. Diese Platte **60** besteht gesamthaft oder mindestens in den Bereichen, aus welchen die Abutments **18** erzeugt werden, aus dem Material der Abutments **18** selbst, dient aber auch als Vorrichtung zum Fixieren der entstehenden Abutments **18** während ihrer Herstellung. Die Platte **60** enthält bereits zum Beispiel an mehreren, im dargestellten Beispiel an acht, Stellen je ein Positionierungselement **16**, das auch am fertigen Abutment **18** dessen Positionierungselement **16** bilden wird. Die Positionierungselemente können beliebige Formen bilden, beispielsweise ein Hexagon, ein Octagon, einen Rhombus, einen Ovalzylinder. **Fig. 8A** zeigt als Beispiel vier Varianten **16.1, 16.2, 16.3, 16.4** von rotationsverhindernden Positionierungselementen **16**, die gemäss **Fig. 8B** alle nach Aussen ragen, obwohl im allgemeinen wie in **Fig. 8C** dargestellt alle Positionierungselemente **16** einer Platte **60** gleich ausgebildet sind. Die Positionierungselemente **16** der Platte **60** gemäss **Fig. 8D** sind dagegen im Inneren der Platte **60** angebracht. Die Platte **60** enthält auch die Durchgangsbohrungen **19** und die Auflage für einen Kopf der hier nicht dargestellten Schraube **54**, die später zur Fixierung der fertigen Abutments **18** auf den Implantaten **12** benötigt werden. Wenn Abutments ohne Durchgangsbohrungen hergestellt werden müssen, so sind Platten **60** ohne die Bohrungen **19** zu verwendet. Aus der Platte **60** können somit acht Verbindungsstücke **18** hergestellt werden, die dazu bestimmt sind, auf je einem Implantat befestigt zu werden. Die Formen der fertigen Abutments **18** sind in **Fig. 8D** mit gestrichelten Linien dargestellt.

**Fig. 9A** und **Fig. 9B** zeigen vier fertige Abutments **18**, die aus einer Platte **60** hergestellt worden sind.

**Fig. 10A** und **10B** zeigen, in analoger Darstellung wie **Fig. 9A** und **9B**, wie die Herstellung des individuellen Zahnersatzes **10** weiter rationalisiert werden kann, indem jeweils ein Abutment integral mit einem Gerüst, gegebenenfalls auch integral mit einer Verblendung, als Integralteil **24** ausgebildet und aus demselben Material in einem Bearbeitungsgang hergestellt werden. Besonders rationell ist es, mehrere Integralteile **24** aus einer Platte **70** herzustellen.

Die Abutments **18** und Integralteile **24**, die aus Platten **60** bzw. **70** hergestellt werden, werden erst am Ende ihrer Bearbeitung von den Platten getrennt, falls die Platten **60** bzw. **70** direkt eingespannt werden. Alternativ können die Platten **60** bzw. **70** während der Bearbeitung der Abutments **18** bzw. Integralteile **24** mittels nicht dargestellter Hilfsschrauben, die in den Bohrungen **19** aufgenommen werden, auf einer nicht dargestellten Vorrichtung befestigt werden; dadurch sind die entstehenden Abutments **18** bzw. Integralteile **24** bis zum Ende der Bearbeitung gehalten. Abutments bzw. Integralteile aus Platten, die keine Bohrungen **19** aufweisen, können ggfs. nach einem ersten Teil der Bearbeitung eingespannt werden, damit sie bis zum Schluss der Bearbeitung gehalten sind.

Aus einer entsprechenden Platte könnte auch ein Integralteil mit drei Abschnitten hergestellt werden, wobei nur zwei Abschnitte zur Befestigung auf je einem Implantat vorgesehen sind und dazu je ein Positionierungselement und eine Durchgangsbohrung aufweisen, während alle drei Abschnitte als Ersatz für insgesamt drei Zähne vorgesehen wären; hierzu müssten aber die Durchgangsbohrungen im erforderlichen Abstand angebracht werden.

Bisher wurde der Frage der Werkstoffe, aus welchen die Implantate, die Abutments, die Gerüste und die Verblendungen hergestellt werden, keine Beachtung geschenkt. Dennoch ist diese Werkstofffrage von entscheidender Bedeutung. Die verwendeten Werkstoffe sollen möglichst preisgünstig und mit vernünftigem Aufwand bearbeitbar sein, eine genügende Härte, Festigkeit und Elastizität und einen dem ursprünglichen Zahnmaterial ähnlichen thermischen Dilationswert aufweisen, um nicht durch mechanische und thermische Beanspruchungen beeinträchtigt zu werden, chemisch resistent sein gegen alle in der Mundhöhle anzutreffenden körpereigenen, zu verspeisenden und bei der Zahnreinigung benutzten Stoffe, biokompatibel sein und die sichtbaren Bereiche des Zahnersatzes, also insbesondere die Aufbauteile, sollten dem ursprünglichen Zahn optisch möglichst ähnlich sein. Ferner ist es erwünscht, dass die verschiedenen Bestandteile des individuellen Zahnersatzes aus demselben Werkstoff hergestellt werden, um alle Probleme zu vermeiden, die sich durch Verwendung verschiedener Werkstoffe ergeben können, beispielsweise unterschiedliche thermische Dilatation, Bi-Material-Effekte wie Korrosion durch unterschiedliche Lage benachbarter Werkstoffe in der elektrischen Spannungsreihe und allzu unterschiedliche Lebensdauer.

Je nach Fall erweisen sich verschiedenen Werkstoffe als besonders geeignet zur Herstellung von Bestandteilen des individuellen Zahnersatzes. Geeignete Werkstoffe sind beispielsweise Metalle, Keramiken, Gläser und Kunststoffe. Die Bearbeitungsanlage, mittels welcher die CAM-Bearbeitung beispielsweise von Abutments durchgeführt wird, sollte daher zur Bearbeitung dieser Werkstoffe fähig sein.

Bezüglich der Techniken, welche zur Herstellung beziehungsweise Bearbeitung der verschiedenen Bestandteile des individuellen Zahnersatzes eingesetzt werden, bestehen Einschränkungen. Insbesondere sind Gussverfahren und Erosionsverfahren, wie sie herkömmlicherweise häufig durchgeführt werden, zur Herstellung nicht unbedingt geeignet, da infolge grosser Temperaturdifferenzen Spannungen oder Materialveränderungen auftreten können. Aus medizinischen Gründen sind insbesondere unkontrollierbare Materialveränderungen zu vermeiden. Für die serienmässige Herstellung von Rohlingen für Implantate und Abutments können, neben anderen Verfahren, problemlos auch Guss- und Erosionsverfahren eingesetzt werden.

Bei Durchführung des erfindungsgemässen Verfahrens lassen sich alle oben erwähnten Anforderungen betreffend der Werkstoffe für den individuellen Zahnersatz erfüllen. Insbesondere können weniger geeignete Gussverfahren vermieden werden und die Bearbeitungsanlage zur Durchführung der CAM-Prozedur kann alle in Frage kommenden Werkstoffe bearbeiten.

Die einzelnen Schritte eines Beispiels des gesamten Verfahrens der Herstellung und Eingliederung eines individuellen Zahnersatzes werden durch die schematischen Darstellungen der **Fig. 11** **und** **12** verdeutlicht.

In **Fig. 11** sind die erfindungsgemässen Schritte durch einen strichpunktierten Rahmen **Q** eingegrenzt. Das Arbeitsmodell **40**, je nach durchgeführten Verfahrensschritten mit den Bestandteilen **42, 18, 20, 22** des individuellen Zahnersatzes **10** beziehungsweise mit dem Hilfselement **44** bestückt, ist stets als Kreis dargestellt, die einzelnen Teile der Einrichtung, welche zur Durchführung des Verfahrens benutzt werden, nämlich der Scanner **SCAN**, die EDV-Einheit **EDV** sowie die CAD/CAM-Anlage **CC**, sind als Parallelogramme dargestellt und durch einen Rahmen **P** zusammengefasst, und die aus der EDV-Einheit **EDV** und der CAD/CAM-Anlage **CC** resultierenden Bestandteile **12, 16, 18, 20, 22** des individuellen Zahnersatzes **10** sowie das Hilfselement beziehungsweise Vermessungsteil **44** und Daten für deren Montage sind durch Rechtecke dargestellt. Ausserhalb des Rahmens **Q** angegebene Teile beziehungsweise Verfahrensschritte, nämlich der mit dem individuellen Zahnersatz **10** zu versehende Kieferbereich **30** des Patienten, das danach gefertigte Negativmodell **32,** der mit den Implantaten **12** versehene Kieferbereich **30.1** und der mit dem individuellen Zahnersatz **10** versehene Kieferbereich **30.2** des Patienten gehören nicht zur Erfindung. Im übrigen ist **Fig.11** selbsterklärend.

**Fig. 12** zeigt in anschaulicher Weise die verschiedenen Herstellungsvarianten sowie die Anfangs-, Zwischen- und Endstadien des Zahnersatzes mit Ausnahme der Implantate. Benutzt werden die Erfassungseinrichtung **SCAN**, die EDV-Anlage **EDV** und die CAD/CAM-Anlage **CC**.

Das Ausgangsmaterial für die Abutments **18** bilden entweder die Abutment-Rohteile **17,** die während ihrer Bearbeitung an den Platten **50** befestigt werden, oder die aus AbutmentMaterial bestehenden Platten **60**. Auf die Abutments **18** wird anschliessend die Suprakonstruktion **21** montiert; diese kann aus einem Teil oder aus zwei Teilen, nämlich dem Gerüst **20** und der Verblendung **22**, bestehen.

Das Ausgangsmaterial für die Integralteile **24**, die entweder das Abutment und das Gerüst oder das Abutment und das Gerüst und die Verblendung integrieren, bildet die Platte **70**.

### Bezugszeichenliste

- **10**: Zahnersatz
- **12**: Implantat

- **14**: 1. Positionierungselement (an Implantat)
- **14.1**: 1. Positionierungselement (an Manipulierimplantat)
- **14.2**: 1. Positionierungselement (an Platte **50**)

- 15: Positionierungsvorrichtung
- **16**: 2. Positionierungselement an Verbindungsstück beziehungsweise an Platten **60, 70**
- **17**: Abutment-Rohteil (= *Abutment* Standard)
- **18**: Abutment
- **19**: Durchgangsbohrung in **18**
- **20**: Gerüst
- 21: Suprakonstruktion = **20 + 22** *aus einem Material gefertigt **xxxx***
- **22**: Verblendung

- **24**: Integralteil
- **24.1**, **24.2**, **24.3**: Abschnitte von **24**

- **30**: Kieferbereich
- **30.1**: Kieferbereich mit Implantaten
- **30.2**: Kieferbereich mit Zahnersatz

- **32**: Kieferknochen
- **34**: Zahnfleisch

- **40**: Arbeitsmodell
- **42**: Manipulierimplantat
- **44**: Hilfselement
- **45**: Markierung an **44**
- **46**: Fixationsschlüssel

- **50**: Platte (=Vorrichtung) *für Standard- Abutment*
- **52**: Gewindebohrung
- **54**: Schraube

- **60**: Platte = *Rohling mit eingearbeiteten Positionierungselement mit Schraubenkanal*

- **70**: Platte = Rohling für Kronen und Brückengerüste 20 oder für komplette Suprakonstruktionen 20 + 21

- **T1**, **T2**, **T3**: Stelle früherer Zähne in **30**
- **M1**, **M2**: Stellen wo Manipulierimplantat gesetzt ist, in **40**

- **SCAN**: Scanner
- **EDV**: EDV-Anlage
- **CC**: CAD/CAM-Anlage

- **P**: 1. Rahmen in ***Fig. 10***
- **Q**: 2. Rahmen in ***Fig. 10***

- **E**: Einschieberichtung

- **BAD**: Basis-Daten
- **ID**: Implantat-Daten
- **VD**: Verbindungsstück-Daten
- **ED**: Einschiebe-Daten
- **GD**: Gerüst-Daten
- **BD**: Verblendungs-Daten
- **FD**: Fixierungsschlüssel-Daten

## Patentansprüche

1. Verfahren zur Herstellung eines individuell gefertigten implantat-gestützten Zahnersatzes (10), insbesondere aus beliebigen, auch biokompatiblen Werkstoffen und insbesondere mit Hilfe der CAD/CAM Technik,
- wobei in einem Arbeitsmodell (40) eines mit dem Zahnersatz (10) zu versehenden Kieferbereichs (30) mindestens ein Manipulierimplantat (42) angebracht wird, welches relativ zum Arbeitsmodell (40) eine Lage einnimmt, die der Lage eines im Kieferbereich (30) implantierten Implantates (12), das an seinem distalen Ende ein Positionierungselement (14) aufweist, entspricht,
- wobei zu jedem Implantat (12) ein Abutment (18) hergestellt wird, das dazu bestimmt ist, am Implantat (12) mittels einer Positionierungsvorrichtung (15) positioniert und mittels eines Befestigungselementes befestigt zu werden,
- wobei am Arbeitsmodell (40) eine auf das Abutment (18) passende Suprakonstruktion (21) aufgebaut wird, welche dazu bestimmt ist, im Kieferbereich (30) auf das mindestens eine Abutment (18) geschoben zu werden,
- wobei nach dem Anbringen des mindestens einen Manipulierimplantates (42) im Arbeitsmodell (40) auf jedem Maniputierimplantat (42) ein Hilfselement (44) angebracht wird, das zusammen mit dem Manipulierimplantat (42) die Lage des Implantates (12) im Kieferbereich (30) wiedergibt,
- wobei mittels einer Erfassungsvorrichtung (SCAN) die dreidimensionale Geometrie des Arbeitsmodells (40) mit dem mindestens einen Hilfselement (44) erfasst wird und daraus Basis- Daten (BAD) ermittelt werden, welche die genannte dreidimensionale Geometrie beschreiben,
- wobei aus den genannten Basis-Daten (BAD) Implantat-Daten (ID) ermittelt werden, welche die Tiefe, die Neigung und die Winkellage des Implantates (12) im Kieferbereich (30) definieren,
- wobei aus den Implantat-Daten (ID) Abutment- Daten (AD) ermittelt werden, welche das herzustellende Abutment (18) definieren, und
- wobei die Herstellung des Abutments (18) unter Berücksichtigung der Abutment-Daten (AD) mittels einer CAD/CAM-Anlage (CC) erfolgt,
**dadurch gekennzeichnet,**
- **dass** das Hilfselement (44) nicht zu dem Zahnersatz gehört und
- **dass** unter Benutzung der Basis-Daten (BAD) Einschubdaten (ED) ermittelt werden, die eine Einschubrichtung (E) definieren, in welcher ein Gerüst (20) oder eine das Gerüst (20) und eine Verblendung (22) umfassende Suprakonstruktion (21) auf ein oder mehrere Abutments (18) geschoben wird.

2. Verfahren nach Anspruch 1,
**dadurch gekennzeichnet,**
**dass** das Abutment (**18**) unter Benutzung der Abutment-Daten (**AD**) aus einem einzelnen Abutment-Rohteil (**17**) oder aus einem Standard-Abutment oder aus einer aus Abutment-material bestehenden Platte (**60**) hergestellt wird.

3. Verfahren nach mindestens einem der obigen Ansprüche,
**dadurch gekennzeichnet,**
**dass** zur Festlegung der Lage des mindestens einen Manipulierimplantates (**42**) das Arbeitsmodell (**40**) benutzt wird, welches aufgrund einer Kieferabformung des, das mindestens eine Implantat (**12**) enthaltenden, Kieferbereiches (**30.1**) hergestellt ist.

4. Verfahren nach mindestens einem der obigen Ansprüche,
**dadurch gekennzeichnet,**
**dass** aus den Basisdaten (**BAD**) und aufgrund von mindestens einem im Arbeitsmodell angebrachten Abutment (**18**) Fixierungs-Daten (**FD**) ermittelt werden.

5. Verfahren nach mindestens einem der obigen Ansprüche,
**dadurch gekennzeichnet,**
**dass** unter Benutzung der Basis-Daten **(BAD)** mittelbar über die Abutment-Daten **(AD)** Gerüstdaten **(GD)** ermittelt werden, aufgrund welcher das Gerüst **(20)** oder die Suprakonstruktion **(21)** mittels der CAD/CAM-Anlage **(CC)** hergestellt wird.

6. Verfahren nach mindestens einem der obigen Ansprüche,
**dadurch gekennzeichnet,**
**dass** unter Benutzung der Basis-Daten **(BAD)** mittelbar über die Abutment-Daten **(AD)** und die Gerüst-Daten **(GD)** Verblendungsdaten **(VD)** ermittelt werden, mittels welchen die Verblendung **(22)** des Gerüstes **(20)** mittels der CAD/CAM-Anlage **(CC)** hergestellt.wird.

7. Verfahren nach mindestens einem der obigen Ansprüche,
**dadurch gekennzeichnet,**
**dass** zur Herstellung einer kronen-oder brückenartigen Suprakonstruktion **(21)** oder Verblendung **(22)** ein Wax up oder eine Bibliothek benutzt wird.

8. Verfahren nach mindestens einem der obigen Ansprüche,
**dadurch gekennzeichnet,**
**dass** das Gerüst (**20**) der Suprakonstruktion (**21**) integral mit dem Abutment **(18)** hergestellt wird.

9. Verfahren nach Anspruch 1,
**dadurch gekennzeichnet,**
**dass** die das Gerüst **(20)** und die Verblendung **(22)** umfassende Suprakonstruktion **(21)** integral, vorzugsweise bei vorherigem Wax up oder aus einer Bibliothek, hergestellt wird.

10. Verfahren nach mindestens einem der obigen Ansprüche,
**dadurch gekennzeichnet,**
**dass** die das Gerüst (**20**) und die Verblendung (**22**) umfassende Suprakonstruktion (**21**) integral mit dem Abutment (**18**) hergestellt wird.

11. Verfahren nach mindestens einem der obigen Ansprüche,
**dadurch gekennzeichnet,**
**dass**, vorzugsweise unter Benutzung der Basis-Daten (**BD**), eine Schablone hergestellt wird, mit deren Hilfe die vorgesehene Lage des inserierten Zahnersatzes überprüft wird.

## Claims

1. Method for producing an individually produced, implant-supported dental prosthesis (10), in particular from arbitrary, also biocompatible materials and in particular with the aid of the CAD/CAM technology:
- whereby at least one manipulation implant (42) is attached in a working model (40) of a jaw region (30) to be provided with the dental prosthesis (10), adopting a position relative to the working model (40) that corresponds to the position of an implant (12) implanted in the jaw region (30) that exhibits a positioning element (14) at its distal end;
- wherein an abutment (18) is produced for each implant (12), this being intended to be positioned at the implant (12) by means of a positioning device (15), and fastened by means of an fastening element;
- wherein a supraconstruction (21) fitting on the abutment (18) is set up on the working model (40), this supraconstruction being intended to be pushed onto the at least one abutment (18) in the jaw region (30);
- wherein, after the attachment of the at least one manipulation implant (42) in the working model (40), an auxiliary element (44) is attached on each manipulation implant (42) that reproduces the position of the implant (12) in the jaw region (30) together with the manipulation implant (42);
- wherein the three-dimensional geometry of the working model (40) with the at least one auxiliary element (44) is detected by means of an acquisition device (SCAN), and from this basic data (BAD) are determined that describe said three-dimensional geometry;
- wherein implant data (ID) are determined from said basic data (BAD) which define the depth, the inclination and the angular position of the implant (12) in the jaw region (30);
- wherein abutment data (AD) are determined from said implant data (ID) which define the abutment (18) to be produced; and
- wherein the production of the abutment (18) is performed by means of a CAD/CAM system (CC), taking into consideration said abutment data (AD);
**characterized in that**
- the auxiliary element (44) does not belong to the dental prosthesis
- insertion data (ED) are determined by using said basic data (BAD) that define an insertion direction (E) in which a framework (20) or a supraconstruction (21) comprising the framework (20) and veneer (22) is pushed onto one or more abutments (18).

2. Method according to claim 1, **characterized in that** the abutment (18) is produced from a single raw abutment part (17) or from a standard abutment or from a plate (60) consisting of abutment material using the abutment data (AD).

3. Method according to at least one of the above claims **characterized in that** the working model (40), which is produced from a jaw impression of the jaw region (30.1) containing the at least one implant (12), is used to define the position of the at least one manipulation implant (42).

4. Method according to at least one of the above claims **characterized in that** fixing data (FD) are determined from the basic data (BAD) and based on at least one abutment (18) attached in the working model.

5. Method according to at least one of the above claims **characterized in that** by using the basic data (BAD) framework data (GD) are indirectly determined through the abutment data (AD), by means of which the framework (20) or the supraconstruction (21) is produced by means of the CAD/CAM system (CC).

6. Method according to at least one of the above claims **characterized in that** by using the basic data (BAD) veneer data (VD) are indirectly determined through the abutment data (AD) and the framework data (GD), by means of which the veneer (22) of the framework (20) is produced by means of the CAD/CAM System (CC).

7. Method according to at least one of the above claims **characterized in that** a wax-up or a library is used to produce a crown-like or bridge-like supraconstruction (21) or veneer (22).

8. Method according to at least one of the above claims **characterized in that** the framework (20) of the supraconstruction (21) is produced integrally with the abutment (18).

9. Method according to claim 1 **characterized in that** the supraconstruction (21) comprising the framework (20) and the veneer (22) is produced integrally, preferably with a previous wax-up or from a library.

10. Method according to at least one of the above claims **characterized in that** the supraconstruction (21) comprising the framework (20) and the veneer (22) is produced integrally with the abutment (18).

11. Method according to at least one of the above claims **characterized in that** a template is produced, preferably using the basic data (BAD), by means of which the intended position of the dental prosthesis is examined.

## Revendications

1. Procédé de fabrication d'une prothèse dentaire (10) soutenue par un implant fabriqué individuellement, en particulier fabriqué dans des matériaux quelconques, aussi biocompatibles, et en particulier à l'aide de la technique CAO/PAO,
- dans lequel, dans un modèle de travail (40) d'une zone de la mâchoire (30) à équiper de la prothèse dentaire, on applique au moins un implant de manipulation (42) qui, par rapport au modèle de travail (40), adopte une position qui correspond à la position d'un implant (12) implanté dans une zone de mâchoire (30) et qui présente au niveau de son extrémité distale un élément de positionnement (14),
- dans lequel, pour chaque implant (12), on fabrique une dent-pilier (18) qui est définie pour être positionnée au niveau de l'implant (12) au moyen d'un dispositif de positionnement (15) et être fixée au moyen d'un élément de fixation,
- dans lequel, au niveau du modèle de travail (40), on élabore une supraconstruction (21) qui s'adapte sur la dent-pilier (18) et qui sert à être poussée dans la zone de la mâchoire sur la dent-pilier (18), au moins au nombre de une,
- dans lequel, après l'application de l'implant de manipulation (42), au moins au nombre de un, dans le modèle de travail (40), on applique sur chaque implant de manipulation (42) un élément auxiliaire (44) qui, avec l'implant de manipulation (42), restitue la position de l'implant (12) dans la zone de la mâchoire (30),
- dans lequel, au moyen d'un dispositif d'acquisition (SCAN), on enregistre la géométrie en trois dimensions du modèle de travail (40) avec l'élément auxiliaire (44), au moins au nombre de un, et on détermine à partir de cela des données de base (BAD) qui décrivent la géométrie en trois dimensions nommée,
- dans lequel, à partir des données de base (BAD) nommées, on détermine les données d'implant (ID) qui définissent la profondeur, l'inclinaison et la position angulaire de l'implant (12) dans la zone de mâchoire (30),
- dans lequel, à partir des données d'implant (ID), on détermine les données de dent-pilier (AD), qui définissent la dent-pilier (18) à fabriquer, et
- dans lequel la fabrication de la dent-pilier (18) se fait en tenant compte des données de dent-pilier (AD) au moyen d'une installation de CAO/PAO (CC),
**caractérisé en ce que** l'élément auxiliaire ne fait pas partie de la prothèse dentaire et, en utilisant les données de base (BAD), on détermine des données d'insertion (ED) qui définissent une direction d'insertion (E) dans
laquelle on pousse sur une ou plusieurs dent-piliers (18) une structure (20) ou une supraconstruction (21) comportant la structure (20) et un revêtement (22).

2. Procédé selon la revendication 1, **caractérisé en ce que** la dent-pilier (18) est fabriquée en exploitant les données de dent-pilier (AD) à partir d'un élément brut de dent-pilier (17) unique ou d'une dent-pilier standard ou d'une plaque (60) composée d'un matériau de dent-pilier.

3. Procédé selon au moins l'une des revendications précédentes, **caractérisé en ce que**, pour fixer la position de l'implant de manipulation (42), au moins au nombre de un, on exploite le modèle de travail (40) qui est fabriqué sur la base d'un moulage de mâchoire de la zone de mâchoire (30.1) contenant au moins un implant (12).

4. Procédé selon au moins l'une des revendications précédentes, **caractérisé en ce que** l'on détermine des données de la fixation (FD) à partir des données de base (BAD) et sur la base d'au moins une dent-pilier (18) appliquée dans le modèle de travail.

5. Procédé selon au moins l'une des revendications précédentes, **caractérisé en ce que**, en exploitant les données de base (BAD), on détermine indirectement, par les données de dent-pilier (AD), des données de structure (GD) sur la base desquelles la structure (20) ou la supraconstruction (21) est fabriquée au moyen de l'installation de CAO/FAO.

6. Procédé selon au moins l'une des revendications précédentes, **caractérisé en ce que**, en exploitant les données de base (BAD), on détermine indirectement, par les données de dent-pilier (AD) et les données de structure (GD), des données de revêtement (VD), au moyen desquelles le revêtement de la structure (20) est fabriqué au moyen d'une installation de CAO/FAO (CC).

7. Procédé selon au moins l'une des revendications précédentes, **caractérisé en ce que**, pour fabriquer une supraconstruction (21) de type couronne ou bridge ou un revêtement (22), on utilise une cire pour inlay ou une substance choisie dans une pharmacothèque.

8. Procédé selon au moins l'une des revendications précédentes, **caractérisé en ce que** la structure (20) de la supraconstruction (21) est fabriquée intégralement avec la dent-pilier (18).

9. Procédé selon la revendication 1, **caractérisé en ce que** la supraconstruction (21) englobant la structure (20) et le revêtement (22) est fabriquée intégralement, de préférence avec une cire existante ou une substance choisie dans une pharmacothèque.

10. Procédé selon au moins l'une des revendications précédentes, **caractérisé en ce que** la supraconstruction (21) englobant la structure (20) et le revêtement (22) est fabriquée intégralement avec la dent-pilier (18).

11. Procédé selon au moins l'une des revendications précédentes, **caractérisé en ce que**, de préférence en exploitant les données de base (BD), l'on fabrique un gabarit à l'aide duquel on vérifie la position prévue de la prothèse dentaire insérée.
